(19) Europäisches Patentamt / European Patent Office / Office européen des brevets

(11) **EP 4 751 644 A1**

(12) **EUROPEAN PATENT APPLICATION**

(43) Date of publication:
03.06.2026 Bulletin 2026/23

(21) Application number: 25219356.0

(22) Date of filing: 28.11.2025

(51) International Patent Classification (IPC):
*A61B 5/05* (2021.01)      *A61B 5/11* (2006.01)
*A61B 5/00* (2006.01)

(52) Cooperative Patent Classification (CPC):
**A61B 5/05; A61B 5/1116; A61B 5/7264;**
A61B 5/1121

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 29.11.2024 CN 202411741546

(71) Applicant: **Beijing Metabot Intelligent Robot Technology Co., Ltd.**
**Beijing 100101 (CN)**

(72) Inventor: **Shao, Ming**
**Beijing, 100101 (CN)**

(74) Representative: **Wang, Bo**
**Panovision IP**
**Ebersberger Straße 3**
**85570 Markt Schwaben (DE)**

(54) **SITTING POSTURE DETECTION METHOD AND APPARATUS BASED ON UWB, DEVICE, AND STORAGE MEDIUM**

(57) Embodiments of the present disclosure provide a sitting posture detection method and apparatus based on UWB, a device and a storage medium, which are applied in the technical field of intelligent analysis. The method comprises steps of: by a UWB signal receiver, receiving and amplifying a pulse signal to obtain a first pulse signal, adjusting the phase of the first pulse signal of a corresponding frame based on the difference between the average phase of a reference pulse signal and the phase of the reference pulse signal of any frame, and using a cascade filter to remove noise and increase the signal-to-noise ratio of the received baseband signal to obtain a second pulse signal; using a sudden change in the amplitude of the second pulse signal and/or Doppler information as a sign of a sitting posture change, and determining target distance and angle information of different sitting postures; and, splicing the target distance and angle information as a joint feature and inputting the joint feature into an SVM classifier to obtain a result of sitting posture detection. In this way, the accurate location of a target object can be accurately tracked, and the sitting posture of an individual can be monitored in real time without involving the user's privacy.

100

Receive a pulse signal by a UWB signal receiver, amplify the pulse signal to obtain a first pulse signal, adjust the phase of the first pulse signal of a corresponding frame based on the difference between the average phase of a reference pulse signal and the phase of the reference pulse signal of any frame, and use a cascade filter to remove the noise of the first pulse signal and increase the signal-to-noise ratio of the received baseband signal to obtain a second pulse signal — S110

Use a sudden change in the amplitude of the second pulse signal and/or Doppler information as a division point of a sitting posture change, and determine target distance information and target angle information generated by different sitting postures during the change process according to the second pulse signal before and after different sitting posture changes — S120

Splice the target distance information and the target angle information as a joint feature, and input the joint feature into an SVM classifier to obtain a result of sitting posture detection — S130

FIG. 1

EP 4 751 644 A1

**Description**

**TECHNICAL FIELD**

The present disclosure relates to the technical field of intelligent analysis, and further relates to the crossing field of Internet of Things and medical health, in particular to a sitting posture detection method and apparatus based on UWB, a device and a storage medium.

**BACKGROUND OF THE PRESENT INVENTION**

As the rhythm of modern life speeds up, sedentary lifestyle has become a daily habit for many people, particularly for the elderly. A long-time incorrect sitting posture is often accompanied by various health risks, such as spinal problem and muscle fatigue. Therefore, sitting posture detection has become a highly concerned health monitoring field.

The existing sitting posture detection methods usually rely on cameras and other devices and are easily interfered by light, shield and other factors, resulting in the delay and inaccuracy of the results of sitting posture detection and possibly involving users' privacy.

**SUMMARY OF THE PRESENT INVENTION**

The present disclosure provides a sitting posture detection method and apparatus based on UWN, a device and a storage medium to solve the technical problem that the results of sitting posture detection obtained by the existing detection methods are delayed and inaccurate and also involve users' privacy.

According to the first aspect of the present disclosure, a sitting posture detection method and apparatus based on UWB is provided. The method includes: receiving a pulse signal by a UWB signal receiver, amplifying the pulse signal to obtain a first pulse signal, adjusting the phase of the first pulse signal of a corresponding frame based on the difference between the average phase of a reference pulse signal and the phase of the reference pulse signal of any frame, and using a cascade filter to remove the noise of the first pulse signal and increase the signal-to-noise ratio of the received baseband signal to obtain a second pulse signal;

using a sudden change in the amplitude of the second pulse signal and/or Doppler information as a division point of a sitting posture change, and determining target distance information and target angle information generated by different sitting postures during the change process according to the second pulse signal before and after different sitting posture changes; and

splicing the target distance information and the target angle information as a joint feature, and inputting the joint feature into an SVM classifier to obtain a result of sitting posture detection, the joint feature being a one-dimensional matrix spliced from the target distance information and the target angle information.

In accordance with the aspect described above and any possible implementation, an implementation is further provided, wherein the pulse signal comprises a pulse signal that is sent by the UWB signal receiver and reflected by a target point.

In accordance with the aspect described above and any possible implementation, an implementation is further provided, wherein the adjusting the phase of the first pulse signal of a corresponding frame based on the difference between the average phase of a reference pulse signal and the phase of the reference pulse signal of any frame comprises:

selecting a reflected pulse signal of a static object as the reference pulse signal;
calculating the difference between the average phase of the reference pulse signal of any sequence and the phase of any frame; and
adjusting the phase of the first pulse signal of a corresponding frame according to the difference.

In accordance with the aspect described above and any possible implementation, an implementation is further provided, wherein the using a cascade filter to remove the noise of the first pulse signal and increase the signal-to-noise ratio of the received baseband signal to obtain a second pulse signal comprises:
inputting the phase-adjusted first pulse signal into a low-pass filter in the cascade filter to remove high-frequency noise, and then inputting into a smoothing filter in the cascade filter to further smooth the signal to increase the signal-to-noise ratio of the received signal to obtain a second pulse signal.

In accordance with the aspect described above and any possible implementation, an implementation is further provided, wherein the determining target distance information comprises:

determining a value range of target distance information r according to the resolution and detection range;

traversing the value of the r at a preset step in the value range, and determining an actual value of r according to the measured flight time, the flight time being the time to propagate the pulse signal from the UWB signal sender to the target point and then reflect the pulse signal back to the UWB signal receiver;

traversing all values of r, and calculating P(r) by the following formula: $P(r) = \left| \sum_{n=1}^{N} \sum_{m=1}^{M} s_{n,m} e^{j2\pi \frac{rt}{c}} \right|$, where $s_{n,m}$ is the actually measured baseband signal data; and

determining the value of r at the maximum of the P(r) as target distance information.

In accordance with the aspect described above and any possible implementation, an implementation is further provided, wherein the determining target angle information comprises:

determining the value ranges of target angle information θ and φ according to the resolution and detection range, the value of θ and the value of φ representing the angles of the projections of the signal received by a two-dimensional antenna array in horizontal and vertical directions, respectively;

combining each value of θ and each value of φ in the value ranges, and

calculating a power value P(r,θ,φ) corresponding to the combination of each value of θ and each value of φ by the following formula:

$$P(r, \theta, \phi) = \left| \sum_{m=1}^{M} \sum_{n=1}^{N} \sum_{t=1}^{T} s_{n,m,t} e^{j2\pi \frac{kr}{c}t} e^{j\frac{2\pi}{\lambda}\sin\,\theta(nd\cos\,\phi + md\sin\,\phi)} \right|$$, where N is the number of antennas at the receiver, M is the number of antennas at the transmitter, and $s_{n,m,t}$ is a signal that is sent by the mth transmitting antenna and received by the nth receiving antenna at moment t; and

determining the value of θ and the value of φ corresponding to the maximum value of the power value P(r,θ,φ) as target angle information.

According to the second aspect of the present disclosure, a sitting posture detection apparatus based on UWB, comprising: a signal processing module, configured to receive a pulse signal by a UWB signal receiver, amplify the pulse signal to obtain a first pulse signal, adjust the phase of the first pulse signal of a corresponding frame based on the difference between the average phase of a reference pulse signal and the phase of the reference pulse signal of any frame, and use a cascade filter to remove the noise of the first pulse signal and increase the signal-to-noise ratio of the received baseband signal to obtain a second pulse signal;

an echo detection module, configured to use a sudden change in the amplitude of the second pulse signal and/or Doppler information as a division point of a sitting posture change, and determine target distance information and target angle information generated by different sitting postures during the change process according to the second pulse signal before and after different sitting posture changes; and

a sitting posture detection module, configured to splice the target distance information and the target angle information as a joint feature, and input the joint feature into an SVM classifier to obtain a result of sitting posture detection, the joint feature being a one-dimensional matrix spliced from the target distance information and the target angle information.

According to the third aspect of the present disclosure, an electronic device is provided. The electronic device comprises a memory and a processor, wherein the memory stores a computer program, and the processor executes the program to implement the methods of the first and/or second aspects as described above.

According to the fourth aspect of the present disclosure, a computer-readable storage medium is provided, on which a computer program is stored. When the program is executed by a processor, the method according to the first and/or second aspects of the present disclosure is implemented.

In this disclosure, it is only necessary to receive a pulse signal at the UWB signal receiver, amplify the pulse signal to obtain the first pulse signal, adjust the phase of the first pulse signal of the corresponding frame based on the difference between the average phase of the reference pulse signal and the phase of any frame of the reference pulse signal, and use a cascade filter to remove the noise of the first pulse signal and increase the signal-to-noise ratio of the received baseband signal to obtain the second pulse signal. When the amplitude and/or Doppler information of the second pulse signal suddenly change, it is considered that the sitting posture has changed, and the target distance information and target angle information generated by different sitting postures during the change process are determined based on the second pulse signal before and after different sitting posture changes. Splicing the target distance and angle information as a joint feature and inputting the joint feature into an SVM classifier to obtain a result of sitting posture detection.

The above method can achieve precise position tracking of the target object, real-time monitoring of the individual's sitting posture, and respond immediately when there is a change in sitting posture, promptly reminding the user of poor sitting posture. And the UWB technology has high-speed data transmission capability, which can achieve real-time data collection and processing. In this way, the accurate location of a target object can be accurately racked, and the sitting posture of an individual can be monitored in real time. When the sitting posture is changed, a response is immediately made to promptly alert the user to a bad sitting posture, without involving the user's privacy

It should be understood that the content described in the SUMMARY is not intended to limit the crucial or important features of the embodiments of the present disclosure and the scope of the present disclosure. Other features of the present disclosure will become understandable from the following description.

## BRIEF DESCRIPTION OF THE DRAWINGS

The above and other features, advantages and aspects of the embodiments of the present disclosure will become apparent with reference to the accompanying drawings and the following detailed description. The accompanying drawings are used for better understanding the scheme, and do not constitute any limitations to the present disclosure. Throughout the accompanying drawings, identical or similar reference numerals indicate identical or similar elements, in which:

FIG. 1 shows a flowchart of a sitting posture detection method based on UWB according to an embodiment of the present disclosure;
FIG. 2 shows a block diagram of a sitting posture detection apparatus based on UWB according to an embodiment of the present disclosure; and
FIG. 3 shows a block diagram of an exemplary electronic device capable of implementing the embodiments of the present disclosure.

## DETAILED DESCRIPTION OF THE PRESENT INVENTION

To make the objectives, technical solutions and advantages of the embodiments of the present disclosure clearer, the technical solutions in the embodiments of the present disclosure will be described clearly and completely with reference to the accompanying drawings in the embodiments of the present disclosure. Apparently, the described embodiments are only some but not all of the embodiments of the present disclosure. All other embodiments obtained on the basis of the embodiments in the present disclosure by a person of ordinary skill in the art without paying any creative effort shall fall into the protection scope of the present disclosure.

In addition, the term "and/or" used herein is merely an association relation describing associated objects, and means that there may be three relations. For example, A and/or B may refer to the following three situations: there exists A alone; there exist both A and B; and, there exists B alone. In addition, the character "/" used herein generally indicates that there is an "or" relationship between associated objects.

In the present disclosure, to solve the problems that the existing sitting posture detection methods usually rely on cameras and other devices and are easily interfered by light, shield and other factors so as to result in the delay and inaccuracy of the results of sitting posture detection and possibly involve users' privacy, a sitting posture detection method based on UWB is provided, including steps of: receiving a pulse signal by a UWB signal receiver, amplifying the pulse signal to obtain a first pulse signal, adjusting the phase of the first pulse signal of a corresponding frame based on the difference between the average phase of a reference pulse signal and the phase of the reference pulse signal of any frame, and using a cascade filter to remove the noise of the first pulse signal and increase the signal-to-noise ratio of the received baseband signal to obtain a second pulse signal; using a sudden change in the amplitude of the second pulse signal and/or Doppler information as a division point of a sitting posture change, and determining target distance information and target angle information generated by different sitting postures during the change process according to the second pulse signal before and after different sitting posture changes; and, splicing the target distance information and the target angle information as a joint feature, and inputting the joint feature into an SVM classifier to obtain a result of sitting posture detection. In this way, the accurate location of a target object can be accurately racked, and the sitting posture of an individual can be monitored in real time. When the sitting posture is changed, a response is immediately made to promptly alert the user to a bad sitting posture, without involving the user's privacy.

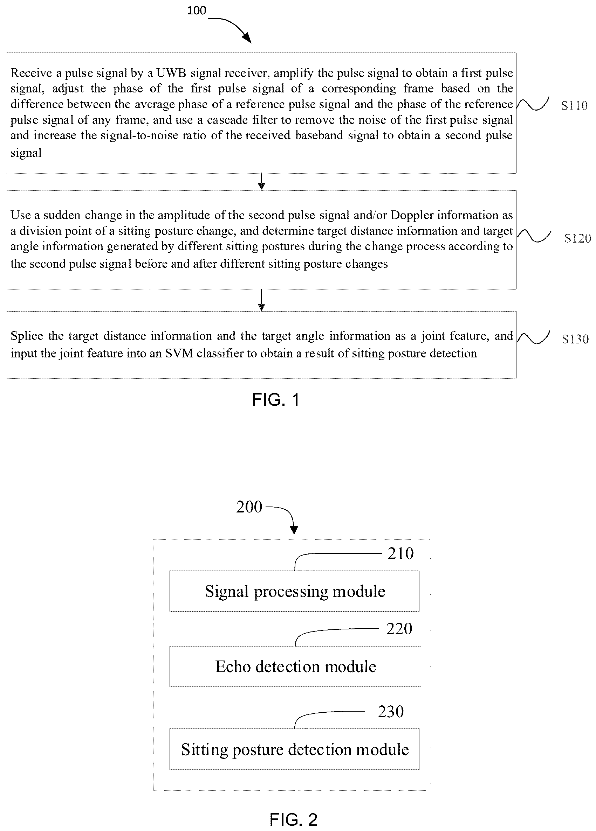

FIG. 1 shows a flowchart of a sitting posture detection method based on UWB 100 according to an embodiment of the present disclosure. As shown in FIG. 1, the sitting posture detection method based on UWB includes the following steps.

In S110, a pulse signal is received by a UWB signal receiver, the pulse signal is amplified to obtain a first pulse signal, the phase of the first pulse signal of a corresponding frame is adjusted based on the difference between the average phase of a reference pulse signal and the phase of the reference pulse signal of any frame, and a cascade filter is used to remove the noise of the first pulse signal and increase the signal-to-noise ratio of the received baseband signal to obtain a second pulse signal.

In some embodiments, the pulse signal includes a pulse signal that is sent by the UWB signal receiver and then reflected.

In some embodiments, a reflected pulse signal of a static object is selected as a reference pulse signal; the difference between the average phase of the reference pulse signal of any sequence and the phase of any frame is calculated; and, the phase of the first pulse signal of a corresponding frame is adjusted according to the difference.

In some embodiments, the phase-adjusted first pulse signal is input into a low-pass filter in the cascade filter to remove high-frequency noise, and then input into a smoothing filter in the cascade filter to further smooth the signal and increase the signal-to-noise ratio of the received baseband signal to obtain a second pulse signal.

In one possible implementation, the cascade filter includes a low-pass filter and a smoothing filter. Firstly, a finite impulse response (FIR) low-pass filter with 26 taps and 1 Hamming window is used. Then, the output of the FIR low-pass filter is smoothed by a five-point-window smoothing filter.

In some embodiments, the UWB signal generator will generate extremely narrow pulses at the nanosecond or microsecond scale, which are transmitted as information carriers in the wireless space. These pulses have extremely short time width, so the spectral range occupied by these pulses is very wide, so that ultra-wideband communication is realized.

In some embodiments, the correlator is a core component in the UWB signal acquisition system. The first pulse signal is sent to the correlator, and the correlator compares and matches the received first pulse signal with the locally generated reference pulse signal by a correlation algorithm to obtain the delay time for transmission of the pulse signal, and determines the distance between the transmitter and the receiver, and processes and analyzes the acquired data to extract useful information such as location and speed.

In one possible implementation, the UWB is placed somewhere in the room, and the antenna faces the detection area to send and receive signals. Specifically, the transmitter sends a signal that can be denoted by

$$s(t) = V_{tx} \exp\left(-\frac{(t-\frac{T_p}{2})^2}{2\sigma_p^2}\right)$$ , where $V_{tx}$ represents the pulse amplitude, $T_p$ represents the signal duration, and

$$\sigma_p = \frac{1}{2\pi B_{-10dB}(\log_{10}(e))^{1/2}}$$ represents the standard deviation determined at -10db bandwidth. After up-conversion, the time-domain transmission signal of the k$^{th}$ frame is $x_k(t) = s(t - kT_s) \cdot \cos(2\pi f_c(t - kT_s))$, where $f_c$ represent the carrier frequency, the operator · is the scalar product, $T_s = \frac{1}{f_p}$ is the duration of the frame, $f_p$ is the pulse repetition frequency,

and $s(t - kT_s) = s(t)$. For simplicity, $t = t' + kT_s$, $t' \in [0, T_s]$, so the transmission signal can be simplified as $x_k(t) = s(t) \cdot \cos(2\pi f_c t)$, and the multipath channel state information (CSI) $h_k(t)$ can be denoted by the following formula:

$$h_k(t) = \sum_{p=1}^{P} \alpha_p \delta\left(t - \tau_p - \tau_p^D(kT_s) - \tau_p^{mD}(kT_s)\right)$$ , where $\alpha_p$ is the propagation attenuation of the p$^{th}$

reflected path signal, $\tau_p$ is the time delay caused during the signal propagation process, $\tau_p^D(kT_s)$ is the time delay caused by a large-range body movement (e.g., walking, jumping, etc.), and $\tau_p^{mD}(kT_s)$ is the time delay caused by a small-range body movement (e.g., the movement of a person's chest due to the ups and downs of breathing). Therefore, the received signal can be represented by:

$$\begin{aligned} y_k(t) &= h_k(t) * x_k(t) \\ &= \sum_{p=1}^{P} \alpha_p \cos\left(2\pi f_c\left(t - kT_s - \tau_p - \tau_p^D(kT_s) - \tau_p^{mD}(kT_s)\right)\right) \\ &\quad \cdot s\left(t - kT_s - \tau_p - \tau_p^D(kT_s) - \tau_p^{mD}(kT_s)\right) + n(t) \end{aligned}$$

where n(t) is a variance, and the operator * is a convolution operation.

In one possible implementation, the ADC of the UWB signal introduces a sampling timing offset (STO) due to an incomplete sampling clock. The disturbance in the signal phase will affect the Doppler and micro-Doppler information of the signal. The Doppler and micro-Doppler are measured by slow time observation $kT_s$. If the reflection comes from a static object, the phase introduced by both Doppler and micro-Doppler is zero. The original RF signal frame (slow time) $y(t)$ has phase noise caused by STO and is reflected from a same static object. The amplitude of the second frame with phase noise has jitter, but ideally, the amplitude should be the same for both frames. In the present invention, due to the presence of phase noise, the static object can be considered as being moving.

The phase of the jittered object is $\Omega_p + \Delta\omega(t - kT_s)$. The phase jitter caused by the STO at the radio receiver is $\Delta\omega(t - kT_s)$, and the phase of the signal reflected by the object is $\Omega_p$. Our goal is to reduce the phase jitter $\Delta\omega(t - kT_s)$.

In one possible implementation, since the UWB is mounted on the ceiling, the maximum peak of reflection is always the floor with the largest radar cross section (RCS). Thus, it is easy to identify this reflection from the reflections from other static objects to correct the phase. Since the change in phase is very small but the motion of the human being is large, the inference result of the neural network is hardly affected. However, this change will make the training stage unstable, and thus leads to long convergence time. The original received baseband signal is destroyed by noise, and the noise will bring errors to the neural network model. If not handled appropriately, random noise will be learned by the neural network, so that it is inclined to over-fitting. Therefore, the cascade filter is used to remove noise and increase the signal-to-noise ratio of the received baseband signal.

In S120, a sudden change in the amplitude of the second pulse signal and/or Doppler information is used as a division point of a sitting posture change, and target distance information and target angle information generated by different sitting postures during the change process are determined according to the second pulse signal before and after different sitting posture changes.

In some embodiments, the determining target distance information includes: determining a value range of target distance information r according to the resolution and detection range; traversing the value of the r at a preset step in the value range, and determining an actual value of r according to the measured flight time, the flight time being the time to propagate the pulse signal from the UWB signal sender to the target point and then reflect the pulse signal back to the UWB signal receiver; and, traversing all values of r, and calculating P(r) by the following formula:

$$P(r) = \left| \sum_{n=1}^{N} \sum_{m=1}^{M} s_{n,m} e^{j2\pi\frac{rt}{c}} \right|$$, where $s_{n,m}$ is the actually measured baseband signal data; and, determining the value of r at the maximum of the P(r) as target distance information.

In some embodiments, the determining target angle information includes: determining the value ranges of target angle information $\theta$ and $\phi$ according to the resolution and detection range, the value of $\theta$ and the value of $\phi$ representing the angles of the projections of the signal received by a two-dimensional antenna array in horizontal and vertical directions, respectively; combining each value of $\theta$ and each value of $\phi$ in the value ranges, and calculating a power value $P(r,\theta,\phi)$ corresponding to the combination of each value of $\theta$ and each value of $\phi$ by the following formula:

$$P(r,\theta,\phi) = \left| \sum_{m=1}^{M} \sum_{n=1}^{N} \sum_{t=1}^{T} s_{n,m,t} e^{j2\pi\frac{kr}{c}t} e^{j\frac{2\pi}{\lambda}\sin\theta(nd\cos\phi + md\sin\phi)} \right|$$, where N is the number of antennas at the receiver, M is the number of antennas at the transmitter, and $s_{n,m,t}$ is a signal that is sent by the $m^{th}$ transmitting antenna and received by the $n^{th}$ receiving antenna at moment t; and, determining the value of $\theta$ and the value of $\phi$ corresponding to the maximum value of the power value $P(r,\theta,\phi)$ as target angle information.

In S130, the target distance information and the target angle information are spliced as a joint feature, and the joint feature is input into an SVM classifier to obtain a result of sitting posture detection, the joint feature being a one-dimensional matrix spliced from the target distance information and the target angle information.

In some embodiments, five sitting postures, i.e., normal sitting posture, leaning left, leaning right, leaning forward and leaning backward, can be distinguished according the distance and angle characteristics.

It should be noted that, for simplicity of description, the above method embodiments are all described as a series of act combinations; however, it should be understood by those skilled in the art that the present disclosure is not limited to the described act order because some steps may be performed in other orders or simultaneously according to the present disclosure. Moreover, it should be understood by those skilled in the art that the embodiments described herein are all optional embodiments and the involved acts and modules are not necessary for the present disclosure.

The method embodiments have been described above, and the scheme of the present disclosure will be further described below by apparatus embodiments.

FIG. 2 shows a block diagram of a sitting posture detection apparatus based on UWB according to an embodiment of the present disclosure. As shown in FIG. 2, the apparatus 200 includes:

a signal processing module 210, configured to receive a pulse signal by a UWB signal receiver, amplify the pulse signal to obtain a first pulse signal, adjust the phase of the first pulse signal of a corresponding frame based on the difference between the average phase of a reference pulse signal and the phase of the reference pulse signal of any frame, and use a cascade filter to remove the noise of the first pulse signal and increase the signal-to-noise ratio of the received baseband signal to obtain a second pulse signal;

an echo detection module 220, configured to use a sudden change in the amplitude of the second pulse signal and/or Doppler information as a division point of a sitting posture change, and determine target distance information and target angle information generated by different sitting postures during the change process according to the second pulse signal before and after different sitting posture changes; and

a sitting posture detection module 230, configured to splice the target distance information and the target angle information as a joint feature, and input the joint feature into an SVM classifier to obtain a result of sitting posture detection, the joint feature being a one-dimensional matrix spliced from the target distance information and the target angle information.

It should be clearly understood by those skilled in the art that, for the convenience and conciseness of description, the specific operation processes of the described modules can refer to the corresponding processes in the above method embodiments and will not be repeated here.

In the technical solutions of the present disclosure, the involved acquisition, storage and application of the personal information of the user all conform to the provisions of relevant laws and regulations, without prejudice to public order and good morals.

In accordance with an embodiment of the present disclosure, the present disclosure further provides an electronic device, a readable storage medium and a computer program product.

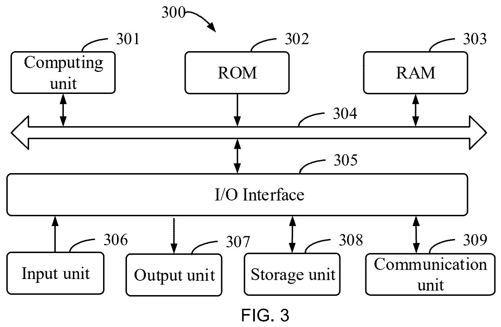

FIG. 3 shows a schematic block diagram of an electronic device 300 capable of implementing the embodiments of the present disclosure. The electronic device is intended to represent various forms of digital computers, such as laptop computers, desktop computers, work benches, personal digital assistants, servers, blade servers, large-scale computers, and other suitable computers. The electronic device may also represent various forms of mobile apparatuses, such as personal digital assistants, cellular phones, smart phones, wearable devices and other similar computing apparatuses. The components illustrated herein, the connections and relations among the components and the functions of the components are only examples, and are not intended to limit the implementations of the present disclosure described and/or claimed herein.

The electronic device 300 includes a computing unit 301, which may execute various appropriate acts and processes according to the computer programs stored in an ROM 302 or the computer programs loaded into an RAM 303 from a storage unit 308. The RAM 303 may also store various programs and data required for the operation of the electronic device 300. The computing unit 301, the ROM 302 and the RAM 303 are connected to each other via a bus 304. An I/O interface 305 is also connected to the bus 304.

A plurality of components connected to the I/O interface 305 in the electronic device 300 includes: an input unit 306, for example, a keyboard, a mouse, etc.; an output unit 307, for example, various types of displays, a loudspeaker, etc.; a storage unit 308, for example, a magnetic disk, an optical disk, etc.; and, a communication unit 309, for example, a network card, a modem, a wireless communication transceiver, etc. The communication unit 309 allows the electronic device 300 to exchange information/data with other devices through a computer network (e.g., the Internet) and/or various telecommunication networks.

The computing unit 301 may be various general-purpose and/or special-purpose processing components having the processing and computing capability. Some examples of the computing unit 301 include, but not limited to: a central processing unit (CPU), a graphics processing unit (GPU), various dedicated artificial intelligence (AI) computing chips, various computing units running machine learning model algorithms, digital signal processors (DSPs), and any suitable processors, controllers, microcontrollers, etc. The computing unit 301 executes the methods and processes described above, for example, the method 100. For example, in some embodiments, the method 100 may be implemented as computer software programs that are tangibly included in a machine-readable medium, e.g., the storage unit 308. In some embodiments, some or all of the computer programs may be loaded into and/or mounted onto the electronic device 300 via the ROM 302 and/or the communication unit 309. When the computer programs are loaded into the RAM 303 and executed by the computing unit 301, one or more steps of the method 100 described above may be executed. Alternatively, in other embodiments, the computing unit 301 may be configured to execute the method 100 in any other suitable ways (e.g., by means of firmware).

Various implementations of the system and technology described above herein may be implemented in digital electronic circuitries, integrated circuitries, field programmable gate arrays (FPGAs), application specific integrated circuits (ASICs), application specific standard products (ASSPs), systems on chip (SOCs), complex programmable logic devices (CPLDs), computer hardware, firmware, software, and/or combinations thereof. These various implementations may include implementations in one or more computer programs. The one or more computer programs may be executed and/or interpreted on a programmable system including at least one programmable processor. The programmable processor may be a special-purpose or general-purpose programmable processor, and may receive data and instructions from a storage system, at least one input device and at least one output device and transmit data and instructions to the storage system, the at least one input device and the at least one output device.

The program codes for implementing the method of the present disclosure may be written in any combination of one or more programming languages. These program codes may be provided to the processors or controllers of general-purpose computers, special-purpose computers or other programmable data processing apparatuses, so that the functions/operations specified in the flowcharts and/or block diagrams are implemented when the program codes are executed by the processors or controllers. The program codes may be executed entirely on a machine, executed partially on a machine, executed partially on a machine and partially on a remote machine as separate software packages, or executed entirely on a remote machine or server.

In the context of the present disclosure, the machine-readable medium may be a tangible medium, which may contain or store programs for use by or use with an instruction execution system, apparatus or device. The machine-readable medium may be a machine-readable signal medium or a machine-readable storage medium. The machine-readable medium may include, but not limited to: electronic, magnetic, optical, electromagnetic, infrared or semiconductor systems, apparatuses or devices, or any suitable combinations thereof. More specific examples of the machine-readable storage medium include: electrical connections based on one or more leads, portable computer disks, hard disks, random access memories (RAMs), read-only memories (ROMs), erasable programmable read-only memories (EPROMs or flash memories), optical fibers, portable compact disc read-only memories (CD-ROMs), optical storage devices, magnetic storage devices or any suitable combination thereof.

In order to provide interaction with a user, the system and technology described herein may be implemented on a computer having: a display device configured to display information to the user; and, a keyboard and a pointing device (e.g., a mouse or a trackball), through which the user can provide an input to the computer. It is also possible to use other types of apparatuses to provide interaction with the user. For example, the feedback provided to the user may be any form of sensory feedback (e.g., visual feedback, auditory feedback or tactile feedback), and the input from the user may be received in any form (including acoustic input, voice input or tactile input).

The system and technology described herein may be implemented in a computing system that include backend components (e.g., as a data server), or a computing system that include middleware components (e.g., an application server), or a computing system that include front-end components (e.g., a user computer having a graphical user interface or web browser, through which the user can interact with the implementations of the system and technology described herein), or a computing system including any combination of backend components, middleware components or front-end components. The components of the system may be connected to each other by any form or medium of digital data communication (e.g., a communication network). Examples of the communication device include: a local area network (LAN), a wide area networks (WAN) and the Internet.

The computing system may include a client and a server. The client and the server are generally far away from each other, and generally interact with each other through a communication network. The relationship between the client and the server is generated by computer programs that are run on the corresponding computers and have a client-server relationship. The server may be a cloud server, a server for a distributed system, or a server that incorporates blockchain.

It should be understood that the steps may be rearranged, added or deleted by using various forms of processes shown above. For example, the steps recorded in the present disclosure may be executed concurrently, sequentially or in a different order as long as the desired results of the technical solutions disclosed in the present disclosure can be achieved, and it will not be limited herein.

The above specific implementations do not constitute any limitations to the protection scope of the present disclosure. It should be understood by those skilled in the art that various modifications, combinations, sub-combinations and substitutions can be made according to the design requirements and other factors. Any modifications, equivalent substitutions and improvements made without departing from the spirit and principle of the present disclosure shall fall into the protection scope of the present disclosure.

## Claims

1. The sitting posture detection method based on UWB, comprising steps of:

   receiving a pulse signal by a UWB signal receiver, amplifying the pulse signal to obtain a first pulse signal, adjusting the phase of the first pulse signal of a corresponding frame based on the difference between the average phase of a reference pulse signal and the phase of the reference pulse signal of any frame, and using a cascade filter to remove the noise of the first pulse signal and increase the signal-to-noise ratio of the received baseband signal to obtain a second pulse signal;
   using a sudden change in the amplitude of the second pulse signal and/or Doppler information as a division point of a sitting posture change, and determining target distance information and target angle information generated by different sitting postures during the change process according to the second pulse signal before and after different sitting posture changes; and
   splicing the target distance information and the target angle information as a joint feature, and inputting the joint feature into an SVM classifier to obtain a result of sitting posture detection, the joint feature being a one-dimensional matrix spliced from the target distance information and the target angle information.

2. The method according to claim 1, wherein the pulse signal comprises a pulse signal that is sent by the UWB signal receiver and reflected by a target point.

3. The method according to claim 1, wherein the adjusting the phase of the first pulse signal of a corresponding frame based on the difference between the average phase of a reference pulse signal and the phase of the reference pulse signal of any frame comprises:

   selecting a reflected pulse signal of a static object as the reference pulse signal;
   calculating the difference between the average phase of the reference pulse signal of any sequence and the phase of any frame; and
   adjusting the phase of the first pulse signal of a corresponding frame according to the difference.

4. The method according to claim 1, wherein the using a cascade filter to remove the noise of the first pulse signal and increase the signal-to-noise ratio of the received baseband signal to obtain a second pulse signal comprises:
   inputting the phase-adjusted first pulse signal into a low-pass filter in the cascade filter to remove high-frequency noise, and then inputting into a smoothing filter in the cascade filter to further smooth the signal to increase the signal-to-noise ratio of the received signal to obtain a second pulse signal.

5. The method according to claim 1, wherein the determining target distance information comprises:

   determining a value range of target distance information r according to the resolution and detection range;
   traversing the value of the r at a preset step in the value range, and determining an actual value of r according to the measured flight time, the flight time being the time to propagate the pulse signal from the UWB signal sender to the target point and then reflect the pulse signal back to the UWB signal receiver;
   traversing all values of r, and calculating P(r) by the following formula:

   $$P(r) = \left| \sum_{n=1}^{N} \sum_{m=1}^{M} s_{n,m} e^{j2\pi \frac{rt}{c}} \right|$$ , where $s_{n,m}$ is the actually measured baseband signal data; and

   determining the value of r at the maximum of the P(r) as target distance information.

6. The method according to claim 1, wherein the determining target angle information comprises:

   determining the value ranges of target angle information $\theta$ and $\phi$ according to the resolution and detection range, the value of $\theta$ and the value of $\phi$ representing the angles of the projections of the signal received by a two-dimensional antenna array in horizontal and vertical directions, respectively;
   combining each value of $\theta$ and each value of $\phi$ in the value ranges, and
   calculating a power value P(r,$\theta$,$\phi$) corresponding to the combination of
   each value of $\theta$ and each value of $\phi$ by the following formula:

   $$P(r,\theta,\phi) = \left| \sum_{m=1}^{M} \sum_{n=1}^{N} \sum_{t=1}^{T} s_{n,m,t} e^{j2\pi \frac{kr}{c}t} e^{j\frac{2\pi}{\lambda}\sin\,\theta(nd\cos\,\phi+md\sin\,\phi)} \right|$$ , where N is the number of antennas at the receiver, M is the number of antennas at the transmitter, and $s_{n,m,t}$ is a signal that is sent by the $m^{th}$ transmitting antenna and received by the $n^{th}$ receiving antenna at moment t; and
   determining the value of $\theta$ and the value of $\phi$ corresponding to the maximum value of the power value P(r,$\theta$,$\phi$) as target angle information.

7. A sitting posture detection apparatus based on UWB, comprising:

   a signal processing module, configured to receive a pulse signal by a UWB signal receiver, amplify the pulse signal to obtain a first pulse signal, adjust the phase of the first pulse signal of a corresponding frame based on the difference between the average phase of a reference pulse signal and the phase of the reference pulse signal of any frame, and use a cascade filter to remove the noise of the first pulse signal and increase the signal-to-noise ratio of the received baseband signal to obtain a second pulse signal;
   an echo detection module, configured to use a sudden change in the amplitude of the second pulse signal and/or Doppler information as a division point of a sitting posture change, and determine target distance information and target angle information generated by different sitting postures during the change process according to the second pulse signal before and after different sitting posture changes; and
   a sitting posture detection module, configured to splice the target distance information and the target angle information as a joint feature, and input the joint feature into an SVM classifier to obtain a result of sitting posture detection, the joint feature being a one-dimensional matrix spliced from the target distance information and the target angle information.

8. An electronic device, comprising:

   at least one processor; and
   a memory in communication with the at least one processor, wherein,
   the memory has instructions stored thereon that can be executed by the at least one processor, and the instructions, when executed by the at least processor, enable the at least one processor to execute the method according to any one of claims 1 to 6.

9. A non-transient computer-readable storage medium having computer instructions stored thereon that are configured to cause a computer to execute the method according to any one of claims 1 to 6.

100

Receive a pulse signal by a UWB signal receiver, amplify the pulse signal to obtain a first pulse signal, adjust the phase of the first pulse signal of a corresponding frame based on the difference between the average phase of a reference pulse signal and the phase of the reference pulse signal of any frame, and use a cascade filter to remove the noise of the first pulse signal and increase the signal-to-noise ratio of the received baseband signal to obtain a second pulse signal — S110

Use a sudden change in the amplitude of the second pulse signal and/or Doppler information as a division point of a sitting posture change, and determine target distance information and target angle information generated by different sitting postures during the change process according to the second pulse signal before and after different sitting posture changes — S120

Splice the target distance information and the target angle information as a joint feature, and input the joint feature into an SVM classifier to obtain a result of sitting posture detection — S130

FIG. 1

200

| Signal processing module — 210 |
| Echo detection module — 220 |
| Sitting posture detection module — 230 |

FIG. 2

300

301
Computing unit

302
ROM

303
RAM

304

305
I/O Interface

306
Input unit

307
Output unit

308
Storage unit

309
Communication unit

FIG. 3

**EUROPEAN SEARCH REPORT**

Application Number

EP 25 21 9356

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | US 10 037 671 B2 (ECHOCARE TECH LTD [IL]) 31 July 2018 (2018-07-31) * figures 1B, 3E, 3F, 10 * * column 19, line 40 - column 20, line 38 * ----- | 1-9 | INV. A61B5/05 A61B5/11 A61B5/00 |
| A | CN 117 133 044 A (UNIV ZHEJIANG SCIENCE & TECH) 28 November 2023 (2023-11-28) * claim 1 * ----- | 1 | |
| A | CN 118 483 698 A (UNIV DALIAN TECH) 13 August 2024 (2024-08-13) * claim 2 * ----- | 1 | |

TECHNICAL FIELDS
SEARCHED      (IPC)

A61B

The present search report has been drawn up for all claims

| Place of search | Date of completion of the search | Examiner |
|---|---|---|
| The Hague | 13 April 2026 | Schwenke, Stephanie |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&  : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 25 21 9356

This annex lists the patent family members relating to the patent documents cited in the above-mentioned European search report.
The members are as contained in the European Patent Office EDP file on
The European Patent Office is in no way liable for these particulars which are merely given for the purpose of information.

13-04-2026

| Patent document cited in search report | | Publication date | Patent family member(s) | | Publication date |
|---|---|---|---|---|---|
| US 10037671 | B2 | 31-07-2018 | EP | 3314591 A1 | 02-05-2018 |
| | | | US | 2016379462 A1 | 29-12-2016 |
| | | | US | 2018330593 A1 | 15-11-2018 |
| | | | WO | 2017002103 A1 | 05-01-2017 |
| CN 117133044 | A | 28-11-2023 | NONE | | |
| CN 118483698 | A | 13-08-2024 | NONE | | |

EPO FORM P0459

For more details about this annex : see Official Journal of the European Patent Office, No. 12/82